# EUROPEAN PATENT APPLICATION

(11) **EP 3 056 125 A1**
(43) Date of publication of application: **17.08.2016**
(21) Application number: 14852281.6
(22) Date of filing: 10.10.2014
(51) Int. Cl.: A47K 7/00

(54) **EAR PICK SEQUENTIALLY DISCHARGING CAPABLE OF DISPOSABLE COTTON SWABS SEQUENTIALLY DISCHARGED THEREFROM**

(30) Priority: 13.10.2013 KR 20130121640
(71) Applicant: Kim, Byoung Chang, Busan 608-817 (KR)
(72) Inventor: Kim, Byoung Chang, Busan 608-817 (KR)
(74) Representative: Ipside
(86) International application number: PCT/KR2014/009522
(87) International publication number: WO 2015/053582

(57) **Abstract**

The present invention relates to an ear pick sequentially discharging disposable cotton swabs, the ear pick including: a guide bar formed penetratedly into the inside thereof and adapted to guide the cotton swabs accommodated in a line thereinto in one direction, without being escaped therefrom; and the cotton swabs accommodated into the guide bar and each having an absorption member disposed on one end thereof to easily pick earwax and an insertion groove formed on the other end thereof, wherein the cotton swabs having relatively short lengths are accommodated in a line into the elongated pen-shaped guide bar, so that even if the size of the guide bar is small so as to improve portability of the ear pick, a large number of the cotton swabs can be accommodated into the guide bar, thus ensuring high portability, and further, the cotton swabs are sequentially discharged from one end of the guide bar through discharge means in a simple way, thus providing many conveniences in use.

## Description

### [Technical Field]

The present invention relates to an ear pick sequentially discharging disposable cotton swabs, and more particularly, to an ear pick sequentially discharging disposable cotton swabs that is configured wherein the cotton swabs having relatively short lengths are accommodated in a line into an elongated pen-shaped guide bar, so that even if the size of the guide bar is small so as to improve portability of the ear pick, a large number of the cotton swabs can be accommodated into the guide bar, thus ensuring high portability, and further, the cotton swabs are sequentially discharged from one end of the guide bar through discharge means in a simple way, thus providing many conveniences in use.

### [Background Art]

Generally, a cotton swab having a wad of cotton mounted on one end thereof is a living item used for a variety of purposes by everyone. The cotton swab is used to pick earwax, repair a woman's makeup, remove foreign materials from a baby's nose, or apply ointment to a wound.

The cotton swab is contained in large quantities into a typical case for containing the cotton swabs, and whenever the use of the cotton swab is needed, it is discharged through a relatively small discharge hole formed on the case. However, it is a little hard to discharge the cotton swab through the relatively small discharge hole, and besides, if a large number of cotton swabs are consumed, the cotton swabs remaining in the case fall sideways, thus making it harder to discharge the cotton swabs from the discharge hole.

Moreover, the length of the cotton swab is generally long to cause the case in which the cotton swabs are contained to be bulky, so that it is hard that the case with the cotton swabs is portable. Accordingly, if the use of the cotton swab is needed at outside, it is really hard to obtain the cotton swab, and accordingly, there are many difficulties in use of the cotton swab.

Furthermore, the cotton swab generally has a shape of a thin bar, so that it cannot be stably taken by a user's hand, and accordingly, it is a little hard to achieve precise movements of the cotton swab in the state wherein the cotton swab is taken by the user's hand, thus disadvantageously causing many inconveniences in use.

### [Disclosure]

### [Technical Problem]

Accordingly, the present invention has been made in view of the above-mentioned problems occurring in the prior art, and it is an object of the present invention to provide an ear pick sequentially discharging disposable cotton swabs that is configured wherein the cotton swabs having relatively short lengths are accommodated in a line into an elongated pen-shaped guide bar, so that even if the size of the guide bar is small so as to improve portability of the ear pick, a large number of the cotton swabs can be accommodated into the guide bar, thus ensuring high portability, and further, the cotton swabs are sequentially discharged from one end of the guide bar through discharge means in a simple way, thus providing many conveniences in use.

It is another object of the present invention to provide an ear pick sequentially discharging disposable cotton swabs wherein a guide bar taken by a user's hand has a generally pen-like shape, and accordingly, the guide bar can be stably taken by the user to achieve precise movements of cotton swabs, so that the cotton swabs can be used for general use thereof as well as for medical uses like wound disinfection or ointment application.

### [Technical Solution]

To accomplish the above-mentioned object, according to the present invention, there is provided an ear pick sequentially discharging disposable cotton swabs, including: a guide bar formed penetratedly into the inside thereof and adapted to guide the cotton swabs accommodated in a line thereinto in one direction, without being escaped therefrom; and the cotton swabs accommodated into the guide bar and each having an absorption member disposed on one end thereof to easily pick earwax and an insertion groove formed on the other end thereof, wherein one end of each cotton swab is laminatedly inserted into the insertion groove of the adjacent cotton swab, so that the cotton swabs are accommodated in a line into the guide bar, while one cotton swab is being exposed outward from one end of the guide bar in such a manner as to be used conveniently by a user.

According to the present invention, preferably, the absorption member of each cotton swab has a shape of a wad of cotton or a spoon.

According to the present invention, preferably, the ear pick further includes a stopper adapted to close the other end of the guide bar in such a manner as to prevent the cotton swabs accommodated into the guide bar from being escaped from the guide bar to the outside.

According to the present invention, preferably, the ear pick further includes discharge means adapted to sequentially discharge the cotton swabs accommodated into the guide bar to one end of the guide bar, so that the cotton swabs accommodated into the guide bar are sequentially discharged to the outside through the manipulation of the discharge means in a simple way.

According to the present invention, preferably, the discharge means includes: a fixing groove formed on one end inner periphery of the guide bar; a fixing hole formed on one end outer periphery of the guide bar in such a manner as to correspond to the fixing groove; and an elastic fastening member having a fixing protrusion formed on one side thereof in such a manner as to be insertedly fixed to the fixing groove, a discharge button formed on the other side thereof in such a manner as to be penetrated into the fixing hole and exposed to the outside, and an oval fastening hole formed in the interior thereof in such a manner as to pressurizingly fasten the outer peripheral surface of the cotton swab exposed outward from the guide bar thereto, whereby if the discharge button of the fastening member is pressed by the user, the other end of the fastening member is pressurized to allow the oval fastening hole of the fastening member to be transformed into the fastening hole having a round shape, so that the cotton swab easily passes through the fastening hole and is discharged to the outside from the guide bar, and if the pressing state of the discharge button is released by the user, the fastening hole transformed to the round shape through the pressurization is returned to the oval shape to pressurize the cotton swab, so that the cotton swab is rigidly fastened to the fastening member.

According to the present invention, preferably, the ear pick further includes a cap 30 adapted to close one end of the guide bar in such a manner as to prevent the cotton swab exposed outward from one end of the guide bar from being exposed to the outside.

According to the present invention, preferably, the ear pick further includes at least two or more guides disposed on the inner peripheral surface of the guide bar in a longitudinal direction of the guide bar to stably guide and convey the cotton swabs accommodated into the guide bar.

### [Advantageous Effects]

According to the present invention, the ear pick according to the present invention is configured wherein the cotton swabs having relatively short lengths are accommodated in a line into the elongated pen-shaped guide bar, so that even if the size of the guide bar is small so as to improve portability of the ear pick, a large number of the cotton swabs can be accommodated into the guide bar, thus ensuring high portability, and further, the cotton swabs are sequentially discharged from one end of the guide bar through the discharge means in a simple way, thus providing many conveniences in use.

Furthermore, the guide bar taken by the user's hand has the general pen-like shape, and accordingly, the guide bar can be stably taken by the user to achieve the precise movements of the cotton swabs, so that the cotton swabs can be used for general use thereof as well as for medical uses like wound disinfection or ointment application.

### [Description of Drawings]

FIG.1 is a perspective view showing an ear pick sequentially discharging disposable cotton swabs according to the present invention.
FIG.2 is a separate perspective view showing the ear pick sequentially discharging disposable cotton swabs of FIG.1.
FIG.3 is a sectional view taken along line A-A of FIG.1.
FIGS.4a to 4e are sectional and perspective views showing the use states of the ear pick sequentially discharging disposable cotton swabs of FIG.1.

### [Mode for Invention]

Hereinafter, an explanation on an ear pick sequentially discharging disposable cotton swabs according to the present invention will be in detail given with reference to the attached drawing.

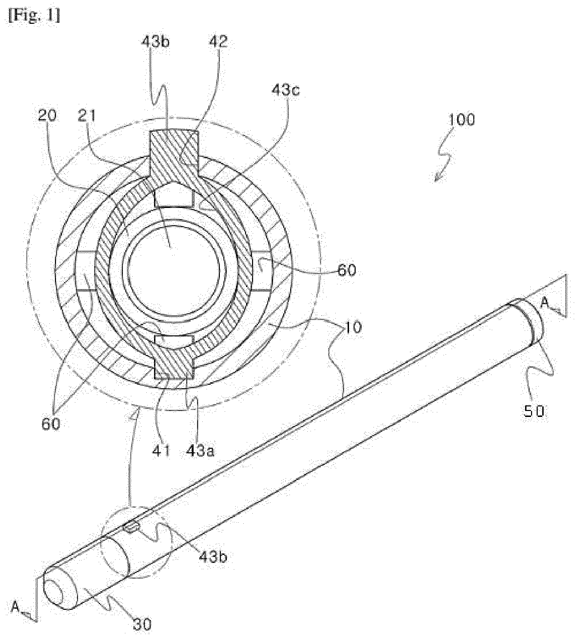

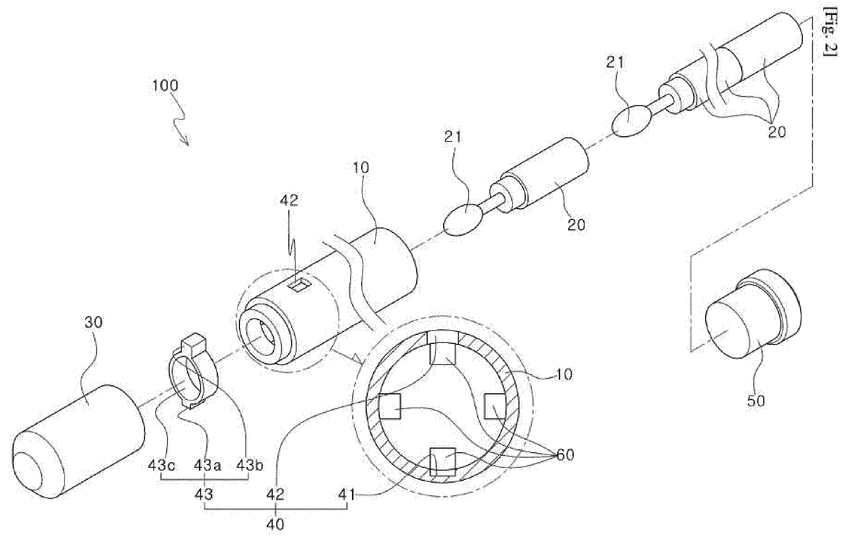

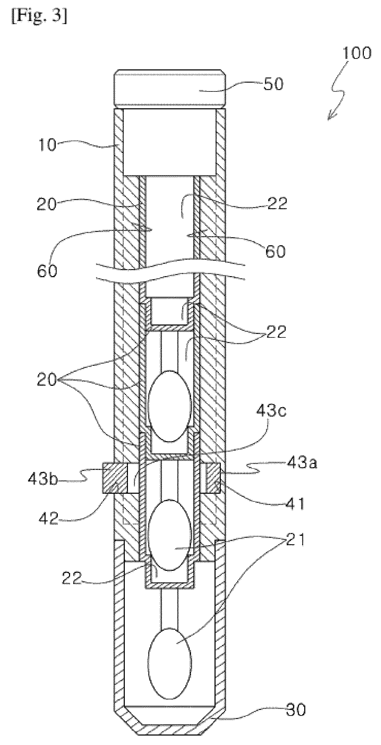

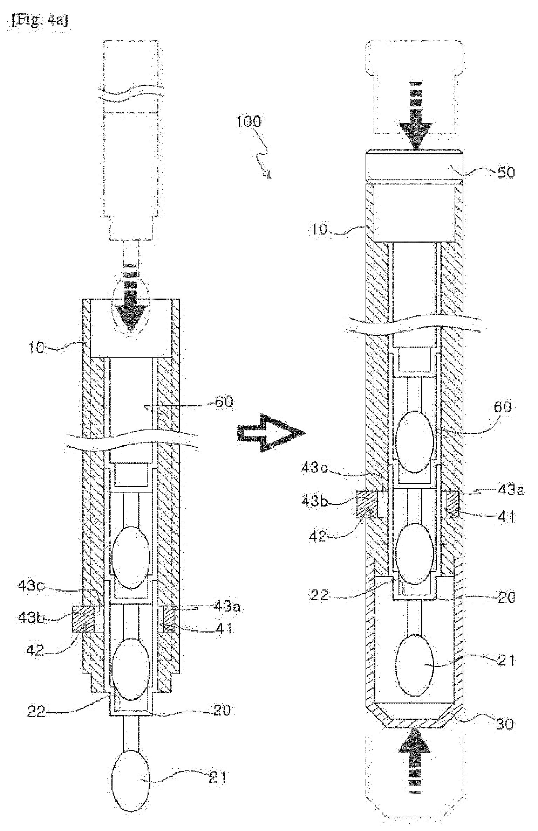

FIGS.1 to 4e show an ear pick sequentially discharging disposable cotton swabs according to the present invention, wherein FIG.1 is a perspective view showing an ear pick sequentially discharging disposable cotton swabs according to the present invention, FIG.2 is a separate perspective view showing the ear pick sequentially discharging disposable cotton swabs of FIG.1, FIG.3 is a sectional view taken along line A-A of FIG.1, and FIGS.4a to 4e are sectional and perspective views showing the use states of the ear pick sequentially discharging disposable cotton swabs of FIG.1.

As shown, an ear pick 100 sequentially discharging disposable cotton swabs according to the present invention largely includes a guide bar 10 and the cotton swabs 20.

As shown in FIGS.1 to 3, the guide bar 10 is formed penetratedly into the inside thereof and adapted to guide the cotton swabs 20 accommodated in a line thereinto in one direction, without being escaped therefrom.

The guide bar 10 of the ear pick 100 sequentially discharging disposable cotton swabs according to the present invention has a shape of an elongated pen-like bar in such a manner as to be stably taken by a user, thus allowing the precise movements of the cotton swabs exposed outward from one end thereof to be induced to provide the conveniences in use.

As shown in FIGS.1 to 3, the cotton swabs 20 are accommodated plurally into the guide bar 10, and each cotton swab 20 has an absorption member 21 disposed on one end thereof to easily pick earwax and an insertion groove 22 formed on the other end thereof.

That is, as shown in FIG.2, one end of each cotton swab 20 is laminatedly inserted into the insertion groove 22 of the adjacent cotton swab 20 thereto, so that the plurality of cotton swabs 20 is accommodated in a line into the guide bar 10. At this time, one cotton swab 20 is exposed outward from one end of the guide bar 10, and accordingly, the exposed cotton swab 20 is conveniently used in the state where the guide bar 10 is taken by the user.

The absorption member 21 of the ear pick 100 according to the present invention is defined as a wad of cotton used to pick earwax, repair a woman's makeup, remove foreign materials from a baby's nose, or apply ointment to a wound, but of course, the absorption member 21 may be extended from the end of the cotton swab 20 to a shape of a spoon, thus being used only for the ear pick.

On the other hand, as shown in FIGS.1 to 3, the ear pick 100 according to the present invention further includes a stopper 50 adapted to close the other end of the guide bar 10 in such a manner as to prevent the cotton swabs 20 accommodated into the guide bar 10 from being escaped from the guide bar 10, and the formation of the stopper 50 improves the structural stability of the ear pick 100.

Further, as shown in FIGS.1 to 3, the ear pick 100 according to the present invention further includes a cap 30 adapted to close one end of the guide bar 10 in such a manner as to prevent the cotton swab 20 exposed outward from one end of the guide bar 10 from being exposed to the outside, and accordingly, the outwardly exposed cotton swab 20 is not contaminated by external environments to induce the sanitary use of the cotton swabs 20.

Furthermore, as shown in FIGS.1 to 3, the ear pick 100 according to the present invention further includes discharge means 40 adapted to sequentially discharge the plurality of cotton swabs 20 accommodated into the guide bar 10 to one end of the guide bar 10, and the discharge means 40 includes a fixing groove 41 formed on one end inner periphery of the guide bar 10, a fixing hole 42 formed on one end outer periphery of the guide bar 10 in such a manner as to correspond to the fixing groove 41, and an elastic fastening member 43 having a fixing protrusion 43a formed on one side thereof in such a manner as to be insertedly fixed to the fixing groove 41, a discharge button 43b formed on the other side thereof in such a manner as to be penetrated into the fixing hole 42 and exposed to the outside, and an oval fastening hole 43c formed in the interior thereof in such a manner as to pressurizingly fasten the outer peripheral surface of the cotton swab 20 exposed outward from the guide bar 10 thereto.

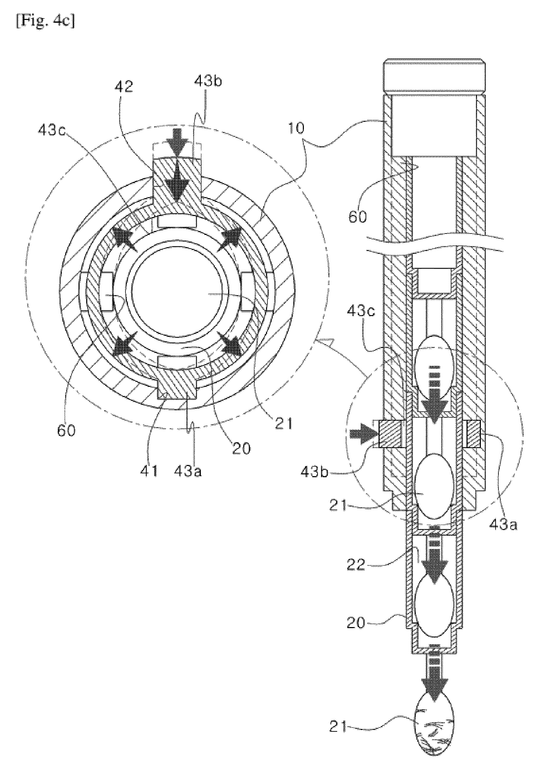

That is, as shown in FIG.4c, if the discharge button 43b of the fastening member 43 is pressed by the user, the other end of the fastening member 43 is pressurized to allow the oval fastening hole 43c of the fastening member 43 to be transformed into the fastening hole 43c having a round shape, so that the cotton swab 20 easily passes through the fastening hole 43c and is discharged to the outside from the guide bar 10.

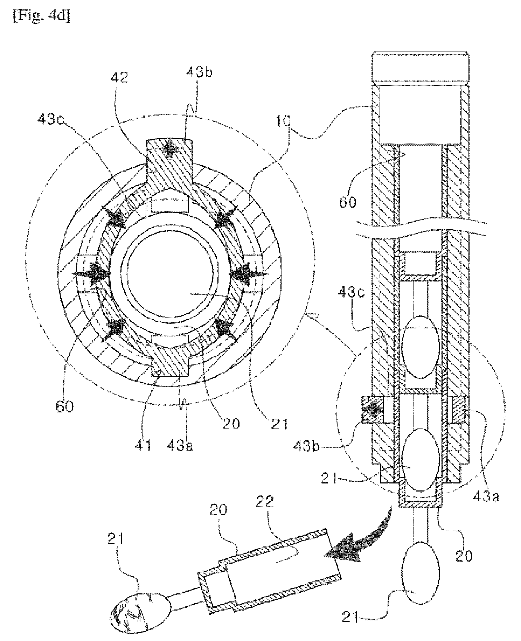

As shown in FIG.4d, if the pressing state of the discharge button 43b is released by the user, the fastening hole 43c transformed to the round shape through the pressurization is returned to the oval shape to pressurize the cotton swab 20, so that the cotton swab 20 is rigidly fastened to the fastening member 43.

Accordingly, the cotton swabs 20 accommodated into the guide bar 10 can be sequentially discharged to the outside through the manipulation of the discharge means 40 in a simple way.

On the other hand, as shown in FIG.2, the ear pick 100 according to the present invention further includes at least two or more guides 60 disposed on the inner peripheral surface of the guide bar 10 in a longitudinal direction of the guide bar 10 to stably guide and convey the cotton swabs 20 accommodated into the guide bar 10, and accordingly, the formation of the guides 60 improves the structural stability of the ear pick 100.

Accordingly, the ear pick 100 according to the present invention is configured wherein the cotton swabs 20 having relatively short lengths are laminatedly accommodated in a line into the elongated pen-like guide bar 10, so that even if the size of the guide bar 10 is small so as to improve portability, a large number of cotton swabs 20 can be accommodated into the guide bar 10, thus ensuring high portability, and further, the cotton swabs 20 are sequentially discharged from one end of the guide bar 10 through the discharge means 40 in a simple way, thus providing many conveniences in use.

Furthermore, the guide bar 10 taken by the user's hand has the general pen-like shape, and accordingly, the guide bar 10 can be stably taken by the user to allow the precise movements of the cotton swabs 20 to be induced, so that the cotton swabs can be used for general use thereof as well as for medical uses like wound disinfection or ointment application.

Under the above-mentioned configuration, an explanation on the operation of the ear pick 100 according to the present invention is given below.

So as to use the ear pick 100 according to the present invention, first, the plurality of cotton swabs 20 elongatedly laminated to each other in a line are inserted and accommodated into the guide bar 10, as shown in FIG.4a.

At this time, the other end of the guide bar 10 is closed by the stopper 50 to prevent the cotton swabs 20 accommodated into the guide bar 10 from being escaped from the guide bar 10, and one end of the guide bar 10 is closed by the cap 30 to prevent the cotton swab 20 exposed outward from one end of the guide bar 10 from being contaminated by external environments.

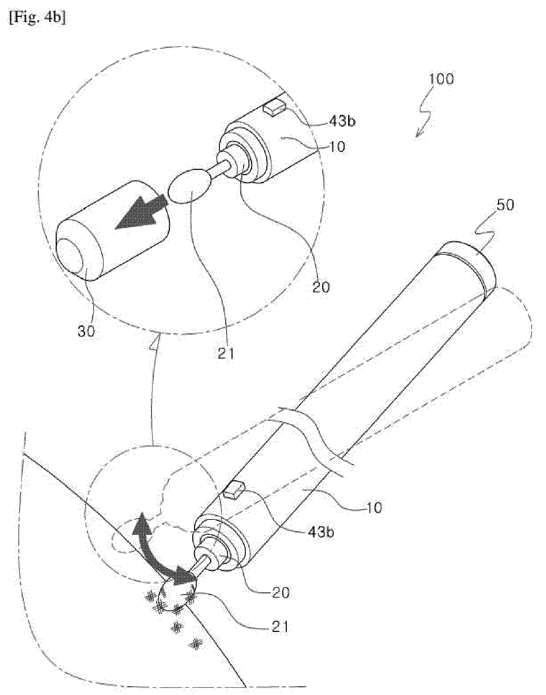

After that, if the use of the cotton swab 20 is needed, the cap 30 is open to simply use the cotton swab 20 exposed outward from one end of the guide bar 10, as shown in FIG.4b.

Next, if the exchange of the cotton swab 20 is needed, the discharge button 43b of the fastening member 43, which is exposed to the outside from the guide bar 10, is pressed by the user, as shown in FIG.4c, and accordingly, the oval fastening hole 43c of the fastening member 43 is transformed into the round fastening hole 43c, so that the cotton swab 20 easily passes through the fastening hole 43c, thus discharging the adjacent cotton swab 20 accommodated into the guide bar 10 to one end of the guide bar 10.

At this time, of course, the cotton swab 20 freely falls and is then discharged to one end of the guide bar 10.

If the adjacent cotton swab 20 is discharged to one end of the guide bar 10, as shown in FIG.4d, the pressing state of the discharge button 43b is released by the user, and accordingly, the fastening hole 43c transformed to the round shape through the pressurization is returned to the oval shape to pressurize the cotton swab 20, so that the cotton swab 20 is rigidly fastened to the fastening member 43.

At this time, the cotton swab 20 after the use is simply separated from the adjacent cotton swab 20 discharged newly to one end of the guide bar 10 and then thrown away.

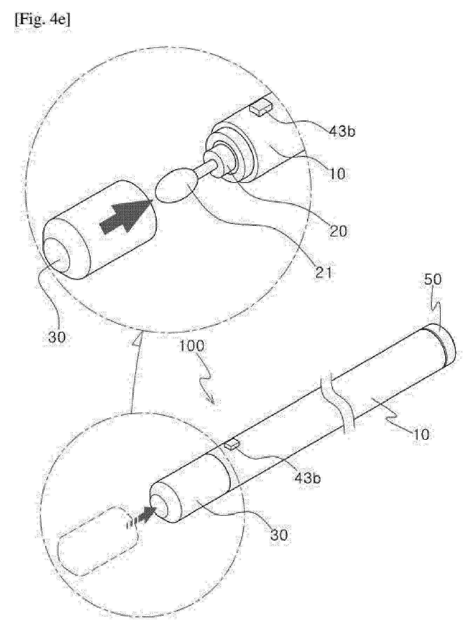

After the use of the ear pick 100 is finished, as shown in FIG.4e, one end of the guide bar 10 is closed by the cap 30 and simply carried or kept.

While the present invention has been described with reference to the particular illustrative embodiments, it is not to be restricted by the embodiments but only by the appended claims. It is to be appreciated that those skilled in the art can change or modify the embodiments without departing from the scope and spirit of the present invention.

## Claims

1. An ear pick 100 sequentially discharging disposable cotton swabs 20, comprising:
a guide bar 10 formed penetratedly into the inside thereof and adapted to guide the cotton swabs 20 accommodated in a line thereinto in one direction, without being escaped therefrom; and
the cotton swabs 20 accommodated into the guide bar 10 and each having an absorption member 21 disposed on one end thereof to easily pick earwax and an insertion groove 22 formed on the other end thereof, wherein one end of each cotton swab 20 is laminatedly inserted into the insertion groove 22 of the adjacent cotton swab 20, so that the cotton swabs 20 are accommodated in a line into the guide bar 10, while one cotton swab 20 is being exposed outward from one end of the guide bar 10 in such a manner as to be used conveniently by a user.

2. The ear pick sequentially discharging disposable cotton swabs according to claim 1, wherein the absorption member 21 of each cotton swab 20 has a shape of a wad of cotton or a spoon.

3. The ear pick sequentially discharging disposable cotton swabs according to claim 1 or 2, further comprising a stopper 50 adapted to close the other end of the guide bar 10 in such a manner as to prevent the cotton swabs 20 accommodated into the guide bar 10 from being escaped from the guide bar 10 to the outside.

4. The ear pick sequentially discharging disposable cotton swabs according to claim 1 or 2, further comprising discharge means 40 adapted to sequentially discharge the cotton swabs 20 accommodated into the guide bar 10 to one end of the guide bar 10, so that the cotton swabs 20 accommodated into the guide bar 10 are sequentially discharged to the outside through the manipulation of the discharge means 40 in a simple way.

5. The ear pick sequentially discharging disposable cotton swabs according to claim 4, wherein the discharge means 40 comprises:
a fixing groove 41 formed on one end inner periphery of the guide bar 10;
a fixing hole 42 formed on one end outer periphery of the guide bar 10 in such a manner as to correspond to the fixing groove 41; and
an elastic fastening member 43 having a fixing protrusion 43a formed on one side thereof in such a manner as to be insertedly fixed to the fixing groove 41, a discharge button 43b formed on the other side thereof in such a manner as to be penetrated into the fixing hole 42 and exposed to the outside, and an oval fastening hole 43c formed in the interior thereof in such a manner as to pressurizingly fasten the outer peripheral surface of the cotton swab 20 exposed outward from the guide bar 10 thereto,
whereby if the discharge button 43b of the fastening member 43 is pressed by the user, the other end of the fastening member 43 is pressurized to allow the oval fastening hole 43c of the fastening member 43 to be transformed into the fastening hole 43c having a round shape, so that the cotton swab 20 easily passes through the fastening hole 43c and is discharged to the outside from the guide bar 10, and if the pressing state of the discharge button 43b is released by the user, the fastening hole 43c transformed to the round shape through the pressurization is returned to the oval shape to pressurize the cotton swab 20, so that the cotton swab 20 is rigidly fastened to the fastening member 43.

6. The ear pick sequentially discharging disposable cotton swabs according to claim 1, further comprising a cap 30 adapted to close one end of the guide bar 10 in such a manner as to prevent the cotton swab 20 exposed outward from one end of the guide bar 10 from being exposed to the outside.

7. The ear pick sequentially discharging disposable cotton swabs according to claim 1, further comprising at least two or more guides 60 disposed on the inner peripheral surface of the guide bar 10 in a longitudinal direction of the guide bar 10 to stably guide and convey the cotton swabs 20 accommodated into the guide bar 10.
